# EUROPEAN PATENT APPLICATION

(11) **EP 0 525 910 A1**
(43) Date of publication of application: **03.02.1993**
(21) Application number: 92202364.3
(22) Date of filing: 30.07.1992
(51) Int. Cl.: C07K 7/10, G01N 33/576, C12N 15/51, C12P 21/08, G01N 33/577, C12Q 1/68, C12Q 1/70, A61K 39/29

(54) **Non-A, non-B peptide**

(30) Priority: 02.08.1991 EP 91201999; 23.10.1991 EP 91202740
(71) Applicant: Akzo Nobel N.V., NL-6824 BM Arnhem (NL)
(72) Inventor: Habets, Winand Johannes Antonius, NL-5232 JB Den Bosch (NL)
(74) Representative: Hermans, Franciscus G.M.

(57) **Abstract**

The invention concerns peptides with react immunochemically with antibodies directed against NANBH.

The invention also relates to a method for the detection of NANBH or anti-NANBH in a test fluid and also to an immunochemical reagent and a test kit to be used when applying the said detection methods.

## Description

The invention relates to a peptide or a fragment thereof and analogues of said peptide and fragments thereof which react immunochemically with antibodies directed against Non-A, Non-B Hepatitis virus (NANBH).

The invention also relates to a method for the detection of NANBH or anti-NANBH in a test fluid and to an immunochemical reagent and a test kit for carrying out said detection method.

Non-A, Non-B hepatitis which may or may not be caused by Hepatitis C Virus (HCV) is a transmissible disease or family of diseases shown to be virus-induced. It can be distinguished from other forms of viral-associated liver diseases, including that caused by the known hepatitis viruses, i.e., hepatitis A virus (HAV), hepatitis B virus (HBV), and delta hepatitis virus (HDV), as well as the hepatitis induced by cytomegalovirus (CMV) or Epstein-Barr virus (EBV). NANBH was first identified in transfused individuals. Transmission from man to chimpanzee and serial passage in chimpanzees provided evidence that Non-A, Non-B Hepatitis is due to a transmissible infectious agent or agents.

Epidemiologic evidence is suggestive that three types of Non-A, Non-B Hepatitis exist: the water-borne epidemic type; the blood or needle associated type; and the sporadically occurring (community acquired) type. However, the number of agents which may be the causative of Non-A, Non-B Hepatitis is unknown.

Clinical diagnosis and identification of Non-A, Non-B Hepatitis has been accomplished primarily by exclusion of other viral markers. Among the methods used to detect putative NANBH antigens and antibodies are agar-gel diffusion, counter-im- munoelectrophoresis, immuno-fluorescence microscopy, immune electron microscopy, radioimmunoassay, and enzyme-linked immunosorbent assay. However, none of these assays has proved to be sufficiently sensitive, specific, and reproducible to be used as a diagnostic test for NANBH.

However, for the development of a specific and sensitive method to enable a reliable diagnosis to be made in various phases of the infection with NANBH it is of great importance to identify immuno-dominant viral epitopes of this type.

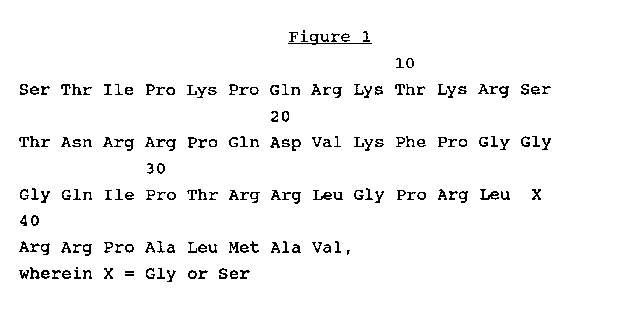

A peptide with 47 amino acids and an amino acid sequence as shown in FIG. 1 has now been found which is surprisingly exceptionally immunochemically reactive with NANBH-antibodies.

The inventive amino acid sequence does not correspond to any published amino acid sequence with regard to NANBH (for example the published sequence by Chiron in EP 388,232).

The invention also comprises fragments of the peptide which are still immunochemically reactive with NANBH-antibodies and also polypeptides comprising the peptide or said fragment thereof.

Analogues or derivatives of the peptide according to FIG. 1 are also included in the invention.

The term "analogues" refers for instance to post-expression modifications of the peptide, for example, glycosylations, acetylations, phosphoryla- tions etc.

Without specifically being incorporated in the claims, it is self-evident that one or more amino acids in the peptides according to the invention can be replaced by other amino acids or amino acid analogues or derivatives without affecting the immunochemical activity of the peptides in question.

The invention also relates to an immunochemical reagent, which reagent comprises at least one of the peptides or fragments thereof according to the invention.

The invention also comprises a method for the detection of antibodies directed against NANBH in a test fluid, in which at least one of these peptides according to the invention is used.

The invention also relates to a method for the detection of NANBH in a test fluid, using at least one of the peptides according to the invention.

The invention also relates to a test kit to be used in an immuno-assay, said test kit containing at least an immunochemical reagent according to the invention.

It is within the scope of this invention to use the new nucleotide sequence or part(s) thereof coding for the amino acid sequence(s) according to the invention so-called primers as the basis of a test to detect NANBH DNA or RNA by a nucleic acid amplification technique for instance the polymerase chain reaction (PCR) or the nucleic acid sequence based amplification (NASBA), as described in USP 4,683,202 and EP 329,822, respectively.

A part of the invention includes a test amplification kit for carrying out an amplification and detection method described above.

Moreover, a peptide or fragment thereof according to the invention can be used in suitable pharmaceutical dosage forms in the prevention and/or treatment of NANB Hepatitis-disease. The preparation of vaccines thus obtained using such a peptide or fragment thereof as active ingredients, can be accomplished by one skilled in the art.

The peptides mentioned above are found to be particularly suitable for use in a diagnostic method for the determination of the presence of NANBH or NANBH-antibodies in a test fluid.

In contrast to the natural NANBH, the peptides according to the invention have the great advantage that these are of a safe non-infectious origin.

The preparation of the peptides according to the invention is effected adapting one of the known organic chemical methods for peptide synthesis or with the aid of recombinant DNA techniques. This latter method involves the preparation of the desired peptide by means of expressing a recombinant polynucleotide with a polynucleotide sequence which is coding for one or more of the peptides in question in a suitable micro-organism as host.

The organic chemical methods for peptide synthesis are considered to include the coupling of the required amino acids by means of a condensation reaction, either in homogeneous phase or with the aid of a so-called solid phase. The condensation reaction can be carried out as follows:
a) condensation of a compound (amino acid, peptide) with a free carboxyl group and protected other reactive groups with a compound (amino acid, peptide) with a free amino group and protected other reactive groups, in the presence of a condensation agent;
b) condensation of a compound (amino acid, peptide) with an activated carboxyl group and free or protected other reaction groups with a compound (amino acid, peptide) with a free amino group and free or protected other reactive groups.

Activation of the carboxyl group can take place, inter alia, by converting the carboxyl group to an acid halide, azide, anhydride, imidazolide or an activated ester, such as the N-hydroxy-succinimide, N-hydroxy-benzotriazole or p-nitrophenyl ester.

The most common methods for the above condensation reactions are: the carbodiimide method, the azide method, the mixed anhydride method and the method using activated esters, such as described in The Peptides, Analysis, Synthesis, Biology Vol. 1-3 (Ed. Gross, E. and Meienhofer, J.) 1979, 1980, 1981 (Academic Press, Inc.).

Preparation of suitable fragments of above-mentioned peptides according to the invention using the "solid phase" is for instance described in J. Amer. Chem. Soc. 85, 2149 (1963) and Int. J. Peptide Protein Res. 35, 161 - 214 (1990). The coupling of the amino acids of the peptide to be prepared usually starts from the carboxyl end side. For this method a solid phase is needed on which there are reactive groups or on which such groups can be introduced. This can be, for example, a copolymer of benzene and divinylbenzene with reactive chloromethyl groups, or a polymeric solid phase rendered reactive with hydroxymethyl or amine-function.

A particulary suitable solid phase is, for example, the p-alkoxybenzyl alcohol resin (4-hydroxymethyl-phenoxy-methyl-copolystyrene-1 % divinylbenzene resin), described by Wang (1974) J. Am. Chem. Soc. 95, 1328. After synthesis the peptides can be split from this solid phase under mild conditions.

After synthesis of the desired amino acid sequence, detaching of the peptide from the resin follows, for example, with trifluoromethanesulphonic acid or with methanesulphonic acid dissolved in trifluoroacetic acid. The peptide can also be removed from the carrier by transesterification with a lower alcohol, preferably methanol or ethanol, in which case a lower alkyl ester of the peptide is formed directly. Likewise, splitting with the aid of ammonia gives the amide of a peptide according to the invention.

The reactive groups which may not participate in the condensation reaction are, as stated, effectively protected by groups which can be removed again very easily by hydrolysis with the aid of acid, base or reduction. Thus, a carboxyl group can be effectively protected by, for example, esterification with methanol, ethanol, tertiary butanol, benzyl alcohol or p-nitrobenzyl alcohol and amines linked to solid support.

Groups which can effectively protect an amino group are the ethoxycarbonyl, benzyloxycarbonyl, t-butoxy-carbonyl or p-methoxy-benzyloxycarbonyl group, or an acid group derived from a sulphonic acid, such as the benzene-sulphonyl or p-toluenesulphonyl group, but other groups can also be used, such as substituted or unsubstituted aryl or aralkyl groups, for example benzyl and triphenylmethyl, or groups such as orthonitrophenyl-sul- phenyl and 2-benzoyl-1-methyl-vinyl. A particularly suitable a-amino-protective groups is, for example, the base-sensitive 9-fluorenylmethoxycarbonyl (Fmoc) group (Carpino & Han (1970) J. Amer. Chem. Soc. 92, 5748).

A more extensive account of possible protecting groups can be found in The Peptides, Analysis, Synthesis, Biology, Vol. 1 - 9 (Eds. Gross, Uden- friend and Meienhofer) 1979 - 1987 (Academic Press, Inc.).

It is necessary also to protect the _{E}-amino group of lysine and advisable for the guanidine group of arginine. Customary protective groups in this connection are a Boc-group for lysine and a Pmc- or Pms- or Mbs-group or Mtr-group for arginine.

The protective groups can be split off by various conventional methods, depending on the nature of the particular group, for example with the aid of trifluoroacetic acid or by mild reduction, for example with hydrogen and a catalyst, such as palladium, or with HBr in glacial acetic acid.

As already indicated above, the peptide according to the invention can likewise be prepared with the aid of recombinant DNA techniques. This possibility is of importance particularly when the peptide is incorporated in a repeating sequence ("in tandem") or when the peptide can be prepared as a constituent of a (much larger) protein or polypeptide. This type of preparation of the peptide therefore likewise falls within the scope of the invention. For this purpose, as a constituent of a recombinant DNA, a polynucleotide is used which codes for the peptide according to the invention.

A polynucleotide of this type, which is coding for the peptide according to the invention, and a recombinant DNA in which this polynucleotide is incorporated likewise fall within the scope of the invention.

In addition the functional derivatives of these peptides, by which are meant in the main:
(a) acid addition salts of the peptides;
(b) amides of the peptides and specifically the C-terminal amides;
(c) esters and specifically C-terminal esters and
(d) N-acyl derivatives, specifically N-terminal acyl derivatives and in particular N-acetyl derivatives,

are also considered as peptides according to the invention.

The peptides or fragments thereof prepared and described above are used to produce antibodies, both polyclonal and monoclonal. Monoclonal antibodies directed against peptides according to the invention can be readily produced by one skilled in the art.

Making monoclonals by hybridomas is well known. Cell fusion, immortal antibody-producing cell lines can be created while also other techniques are available such as direct transformation of B-lymphocytes with oncogenic DNA or transfection with Epstein-Barr Virus.

Antibodies, both monoclonal and polyclonal, directed against peptides according to the invention are very suitable in diagnosis, while those antibodies which are neutralizing are very useful in passive immunotherapy. Especially monoclonal antibodies may be used to raise anti-idiotype antibodies. Techniques for raising anti-idiotype antibodies are known in the art.

Said anti-idiotype antibodies are also useful for prevention and/or treatment of Non-A, Non-B Hepatitis, as well as for the elucidation of important epitopic regions of NANBH-antigens.

A hybridoma cell line producing human monoclonal antibodies were obtained by using the inventive peptide as antigen. Said monoclonal antibodies are directed against the inventive peptides to a high reactivity.

The term "immunochemical reagent" according to the invention usually consists of one more peptides according to the invention and a suitable support or a labelling substance.

Supports which can be used are, for example, the inner wall of a microtest well or a cuvette, a tube or capillary, a membrane, filter, test strip or the surface of a particle such as, for example, a
latex particle, an erythrocyte, a dye sol, a metal sol or metal compound as sol particle, a carrier protein such as BSA or KLH.

Labelling substances which can be used are, inter alia, a radioactive isotope, a fluorescent compound, an enzyme, a dye sol, metal sol or metal compound as sol particle.

In a method for the detection of antibodies directed against NANBH in a test fluid, an immunochemical reagent according to the invention is brought into contact with the test fluid. After which, the presence of immune complexes formed between the peptide and antibodies in the test fluid is detected and by this detection the presence of NANBH antibodies in the test fluid is known and can be determined quantitatively.

Depending on the nature and further characteristics of the immunochemical reagent the immunochemical reaction that takes place is a so called sandwich reaction, an agglutination reaction, a competition reaction or an inhibition reaction.

For the detection of NANBH in a test fluid an immunochemical reagent according to the invention is brought into contact with the test fluid and anti-NANBH after which the presence of immune complexes formed is detected and, from this, the presence of NANBH in a test fluid can be determined.

A particularly suitable method for the detection of NANBH in a test fluid is based on a competition reaction between a peptide according to the invention provided with a labelling substance and a NANBH antigen (present in the test fluid) whereby the peptide and the antigen are competing with the antibody directed against NANBH attached to a solid support.

A test kit according to the invention comprises as an essential constituent an immunochemical reagent as described above. Carrying out a sandwich reaction, for the detection of NANBH antibodies the test kit may comprise, for example, the peptide according to the invention coated to a solid support, for example the inner wall of a microtest well, and either a labelled peptide according to the invention or a labelled anti-antibody. Another sandwich reaction test format is the detection of NANBH antigen whereby monoclonal antibodies according to the invention are coated to a solid support and monoclonal antibodies are used as conjugate.

For instance sandwich reactions are described in our American patents with regard to the enzyme immuno assay viz. RE 31.006 and RE 32.696 (Schuurs et al.).

For carrying out a competition reaction, the test kit may comprise a peptide according to the invention coated to a solid support, and a labelled antibody directed against NANBH preferably a monoclonal antibody directed against said peptide.

In an agglutination reaction the test kit comprises an immunochemical reagent which may comprise a peptide according to the invention coated to particles or sols.

Another embodiment of a test kit is, for example, the use of a labelled peptide according to the invention as immunochemical reagent in a competition reaction with a NANBH antigen to be detected for a binding site on the antibody directed against NANBH, which is coated to a solid support.

### Example I

The peptide (47 amino acids in length) according to the sequence of figure 1 wherein X = Gly is prepared using the above-mentioned solid phase technique and dissolved to 7.5 µg/ml in 100 mM phosphate buffer pH 9.6 and 135 µl of the above peptide solution is placed into each well of a NUNC microtiter plate. Binding of the peptide to the microtiter plate is allowed to proceed overnight at 4° C.

Subsequently the plates are blocked with a solution of 0.05% Tween 20(^{R}) in 0.2 M Tris pH 7.4/0.2 M NaCl for 5 min. at room temperature. Plates are then washed once with 0.2 M Tris pH 7.4/0.2 M NaCI, twice with 0.04 M Tris pH 7.4, at 250 µl per well and dried. For the determination of antibodies specific for Non-A, Non-B Hepatitis virus, the serum samples are diluted in sample diluent (phosphate buffered saline (PBS)/20% normal goat serum/1 % Triton X100) pipetted into the well (100 µl per well) and incubated for 1 h at 37 ° C. After washing the wells with PBS/0.05% Tween 20(^{R}) the bound human antibodies are detected with goat anti-human immunoglobulin labeled with peroxidase (100 µl per well, 1 h at 37 °C) diluted in sample diluent. The plates are washed 4 times with PBS/0.05% Tween 20^{(R)}. TMB is added (100 µl per well) as a substrate for the peroxidase enzyme and the reaction is allowed to proceed for 30 min. at room temperature. The reaction is stopped by adding 100 µl 2M H₂SO₄ to each well. The yellow color is read at 450 nm in an Organon Teknika microelisa reader.

With sera from patients with Non-A Non-B hepatitis extinctions ranging from 1.4 to 2.9 are measured whereas the mean extinction of 18 nomal human sera is 0.208 (standard deviation 0.15).

As a control, the procedure is repeated with two unrelated peptides. In both cases no significant differences are observed in the extinctions obtained with normal human sera and serum samples from patients with NANBH.

## Claims

1. A peptide with amino acid sequence according to figure 1 or a fragment thereof and analogues of said peptide and fragments thereof which are immunochemically reactive with NANBH-antibodies or a polypeptide comprising said peptide or said fragment thereof.

2. Immunochemical reagent comprising a peptide, a fragment or a polypeptide according to claim 1.

3. Vector comprising nucleic acid sequences or fragments thereof coding for the amino acid sequence according to claim 1.

4. A monoclonal antibody directed against a peptide or fragment thereof according to claim 1.

5. Method for the detection of antibodies directed against NANBH in a test fluid, characterized in that an immuno-chemical reagent according to claim 2 is brought into contact with the test fluid and the presence of immune complexes formed between the peptide and antibodies in the test fluid is detected, which is a measure for the presence of NANBH-antibodies in the test fluid.

6. Method for the detection of NANBH in a test fluid characterized in that an immunochemical reagent according to claim 2 is brought into contact with the test fluid and anti-NANBH, whereafter the presence of immune complexes formed is detected and from this the presence of NANBH in the test fluid is determined.

7. Method for the amplification and the detection of a Non-A Non-B Hepatitis nucleic acid sequence in a test fluid using a sequence or fragment thereof coding for the amino acid sequence according to claim 1 as primer(s) in order to perform and to detect a nucleic acid amplification of said Non-A Non-B Hepatitis nucleic acid sequence.

8. Test kit for carrying out an immuno-assay according to claim 5 or 6.

9. Test amplification kit for carrying out an amplification and detection method according to claim 7.
